# EUROPEAN PATENT SPECIFICATION

(11) **EP 0 654 079 B1**
(45) Date of publication and mention of the grant of the patent: **22.03.2000**
(21) Application number: 93916917.3
(22) Date of filing: 01.07.1993
(51) Int. Cl.: C12Q 1/26, C12Q 1/54

(54) **A REAGENT STABILIZED BY BIVALENT METAL SALTS**
REAGENZ STABILISIERT DURCH ZWEIWERTIGE METALLSALZE
REACTIF STABILISE AU MOYEN DE SELS METALLIQUES DIVALENTS

(30) Priority: 02.07.1992 US 907886
(43) Date of publication of application: 24.05.1995
(73) Proprietor: Roche Diagnostics Corporation, Indianapolis, IN 46250 (US)
(72) Inventor: FREITAG, Helmut, E., C., D-6940 Weinheim (DE); WILSEY, Christopher, Douglas, Carmel, IN 46032 (US)
(74) Representative: Jung, Michael, Dr.
(86) International application number: US9306282
(87) International publication number: WO9401544

(56) References cited:
- US-A- 3 929 580
- US-A- 4 427 771
- US-A- 4 457 461
- US-A- 4 748 115
- US-A- 4 892 817
- US-A- 4 946 776
- DATABASE WPI Section Ch, Week 7629 Derwent Publications Ltd., London, GB; Class B04, AN 76-55030X XP002010101 & JP-A-51 063 688 (YATRON LAB INC) , 3 June 1976
- DATABASE WPI Section Ch, Week 8522 Derwent Publications Ltd., London, GB; Class B04, AN 85-130835 XP002010102 & JP-A-60 066 993 (IATRON LABORATORIES) , 17 April 1985

## Description

### FIELD OF THE INVENTION

The invention relates to the chemical analysis of an analyte by the formation of a colored indicator.

### BACKGROUND OF THE INVENTION

A common way of indirectly measuring the amount of analyte in a sample is to involve the analyte in a sequence of reactions that lead to the generation of color, wherein the intensity of the color generated is proportional to the amount of analyte in the sample. A known method of performing this type of chemical assay utilizes reagents that include a phenazine derivative and a tetrazolium salt (the phenazine derivative/tetrazolium salt system). The phenazine derivative/tetrazolium salt system may be schematically depicted by the following scheme for glucose analysis:

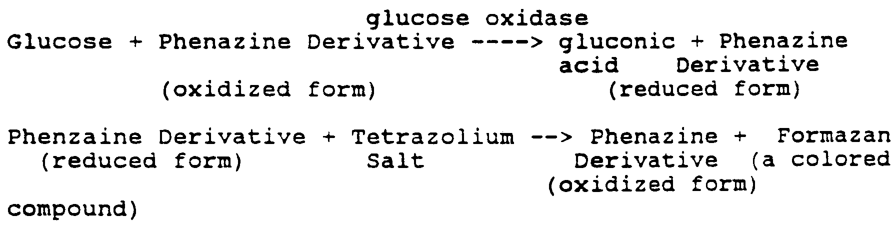

Further illustrations of the phenazine derivative/tetrazolium salt system in chemical analysis may be found in the following references: Findlay et al., U.S. Patent 4,713,327, issued December 15, 1987; Josef et al., U.S. Patent 3,791,988, issued February 12, 1974; Forgione et al., U.S. Patent 3,929,580, issued December 30, 1975; Shigeta et al., U.S. Patent 4,803,158, issued February 7, 1989; Self, U.S. Patent 4,446,231, issued May 1, 1984; and Self, U.S. Patent 4,598,042, issued July 1, 1986.

JP-A 51 063 688 and US-A 4,427,771 disclose reagents comprising an enzyme, a tetrazolium compound or salt, phenazine methosulphate and a bivalent metal salt which is a magnesium salt. This magnesium salt functions only as an enzyme cofactor.

US-A 4,748,115 and US-A 4,946,776 describe compositions comprising a tetrazolium salt and magnesium chloride. These references however are silent with respect to the presence of a phenazine derivative.

US-A 4,892,817 teaches a composition comprising a tetrazolium salt and a water-soluble ferricyanide/ferrocyanide salt or manganate (VII)/manganate (IV) combination. This reference also does not describe the presence of a phenazine derivative. Preferred compositions contain no metal ions other than potassium or sodium.

A problem with the phenazine derivative/tetrazolium salt system is the stability of the phenazine derivative and the tetrazolium salt, particularly when the phenazine derivative and tetrazolium salt are included in a single reagent or a film at pHs above about 5.5. At pHs above about 5.5, photochemical degradation of the phenazine derivative and autoreduction of the tetrazolium salt become significant and interfere with accurate and precise correlation of formazan to the analyte being measured. Because many assays involve the use of enzymes that function best at pHs from about 5.5 to about 7.8, it is desirable to provide means for stabilizing the phenazine derivative/tetrazolium salt system in this pH range.

### SUMMARY OF THE INVENTION

The invention is a stable reagent for the analysis of an analyte. The reagent includes an enzyme, a phenazine derivative, a tetrazolium salt, and a divalent metal salt of sufficient type and in sufficient amount to stablize the reagent.

Phenazine derivative/tetrazolium salt reagent systems become unstable at pHs above about 5.5. However, many enzymes, such as glucose dehyrogenase, hexokinase, and glucose-6-phosphate dehyrogenase, optimally function at pHs above 7. Importantly, the inclusion of the divalent metal salt in the reagent stabilizes the phenazine derivative/tetrazolium salt system at pHs from about 5.5 to about 7.8, thereby permitting the optimal functioning of certain enzymes (such as the enzymes mentioned above) in the assay of an analyte. (At pHs above about 7.8, phenazine derivatives begin to decompose.)

The inventive reagent may be provided in solution form or may be incorporated into a film, a fabric mesh, a glass fiber matrix, or a paper matrix. Stabilizing a film with the inventive reagent is particularly important because ordinary drying of films that include a phenazine derivative and a tetrazolium salt can cause degradation of the reagent.

The invention also describes assay methods utilizing the inventive reagent.

### DETAILED DESCRIPTION OF THE INVENTION

The inventive reagent is useful for the analysis of an analyte (for example, glucose). At a minimum, the reagent includes an enzyme (for example, glucose oxidase), a phenazine derivative (for example, phenazine ethosulfate), a tetrazolium salt (for example, iodonitrotetrazolium chloride (INT) or 3-(4,5-dimethylthiazol-2-yl)-2,5-diphenyl-2H-tetrazolium bromide (MTT)), and a divalent metal salt (such as nickel chloride or manganese sulfate). If the reagent is supplied in solution form, then the reagent will also include water. If the reagent is supplied in a film, then a film forming agent (such as hydroxyethylcellulose and polyvinylpropionate, available from BASF and sold under the trademark PROPIOFAN 70D) will be included. (MTT is very sensitive and was not stalilized in a film.) In a film, water is essentially removed from the reagent by drying the film.

In the reagent, the enzyme must be of sufficient type and in sufficient amount to catalyze the reaction of analyte and phenazine derivative. The kind of enzyme will depend upon the analyte being analyzed. For example, if the analyte is glucose, the enzyme may be glucose oxidase. Other exemplary analyte/enzyme combinations are listed below in Table 1.

**TABLE 1**

| **Analyte** | **Enzyme** |
|---|---|
| Glucose | Glucose Dehydrogenase & Diaphorase |
| Cholesterol | Cholesterol Esterase & Cholesterol Oxidase |
| HDL Cholesterol | Cholesterol Esterase & Cholesterol Oxidase |
| Triglycerides | Lipoprotein Lipase, Glycerol Kinase, & Glycerol-3-Phosphate Oxidase |
| Lactate | Lactate Oxidase |
| Lactate | Lactate Dehydrogenase & Diaphorase |
| Lactate Dehydrogenase | Diaphorase |
| Pyruvate | Pyruvate Oxidase |
| Alcohol | Alcohol Oxidase |
| Bilirubin | Bilirubin Oxidase |

The amount of enzyme will depend upon the speed desired for the assay (the more enzyme added, the faster the test).

The phenazine derivative employed must be of sufficient type and in sufficient amount to catalyze the reduction of the tetrazolium salt. In the context of this invention, phenazine derivatives are used as electron carriers facilitating the reduction of the tetrazolium salt. Examples of phenazine derivatives that may be used are listed below in Table 2.

**TABLE 2**

| Phenazine Derivative: |
|---|
| Phenazine Ethosulfate |
| Phenazine Methosulfate |
| 1-Methoxyphenazins Methosulfate |

Because the phenazine derivative is a non-depleted catalyst in the assay, less than a molar equivalent of phenazine derivative, relative to the analyte being measured, is required in the reagent.

The tetrazolium salt employed must be of sufficient type and in sufficient amount to form a colored indicator when reduced. The reduced tetrazolium salt is a formazan, which is a colored indicator. The amount of this colored indicator formed in an assay is correlated to the amount of analyte in the sample being measured. Examples of the tetrazolium salts that may be used in the reagent are listed below in Table 3.

**TABLE 3**

| Tetrazolium Salts: |
|---|
| MTT (if the reagent is in solution form) (Note: nickel chloride is known to chelate MTT (MTT formazan) and cause a wavelength shift in the MTT formazan.) |
| Iodonitrotetrazolium chloride (INT) |
| Tetranitro blue tetrazolium chloride (TNBT) |

Because there must be at least a molar equivalent of tetrazolium salt with respect to the analyte being measured, a sufficient amount of tetrazolium salt should be included in the reagent to cover the high end of expected concentrations of analyte in the samples being measured.

The focus of this invention is the inclusion of divalent metal salts in the reagent. The divalent metal salts must be of sufficient type and in sufficient amount to stabilize the reagent. Such divalent metal salts include salts with a cation selected from the group consisting of nickel, manganese and magnesium, and an inert anion (an anion that will not chemically react in the assay of the analyte being measured). Specific examples of such divalent metal salts include nickel chloride, manganese sulfate, magnesium sulfate, magnesium chloride, and nickel sulfate. These divalent metal salts may be added individually or in combinations to a reagent to stabilize the reagent. Further, adding a combination of these divalent metal salts to a reagent can sometimes better stabilize the reagent than addition of a single divalent metal salt.

The divalent metal salt cobalt chloride, which imparts a red color to solutions, can stabilize a liquid reagent that includes TRIS buffer (see Table 4). However, cobalt chloride reacts with HEPES buffer (see Table 4) at pH 7.55. Further, cobalt chloride can not be used in a film (or a fabric mesh, a glass fiber matrix, or a paper matrix) that includes a tetrazolium salt because cobalt chloride forms a blue colored complex with water upon drying, thereby interfering with an assay because formazans are blue colored.

When stabilizing a liquid reagent by the addition of divalent metal salts, the molar ratio of divalent metal salts to phenazine derivative should be at least about 30:1. When this ratio was used in a liquid reagent that included magnesium chloride and phenazine ethosulfate, a liquid reagent that did not include a tetrazolium salt was stable for at least 2 hours under ambient laboratory room lighting and temperature conditions. (When no divalent cation was present, this liquid reagent was stable for less than two hours.) It is believed that stability would be about the same for a liquid reagent that also included a tetrazolium salt because it appears that most of the reagent instability is due to instability of the phenazine derivative. (For example, an aqueous solution that includes a buffer and MTT was stable for at least 3 hours.)

Under the same conditions stated above, when the molar ratios of magnesium chloride to phenazine ethosulfate were 30:1, 60:1, 120:1, and 187:1, the reagents were stable for at least 2 hours, 3 hours, 3 days, and 3 days respectively. (Again, it is believed that reagent stability would be about the same if a tetrazolium salt were included.)

Molar ratios of divalent metal salts to phenazine derivative even higher than 187:1 may be used as long as the divalent metal salts are soluble in the reagent and any necessary pH adjustments to the reagent are made. (For example, molar ratios of nickel chloride to phenazine ethosulfate greater than 30:1 require adjustment of the reagent pH because the addition of nickel chloride lowers reagent pH.) Further, the higher the reagent pH, the greater the molar ratio of divalent metal salt to phenazine derivative needs to be in order to stabilize the reagent. The higher molar ratio of divalent metal salt to phenazine derivative is required at higher pHs because degradation of the phenazine derivative and the tetrazolium salt in the reagent become more pronounced at higher pHs.

When a film, a fabric mesh, a glass fiber matrix, and a paper matrix are being stabilized, the ratio of divalent metal salt to phenazine derivative should be at least about 15:1 when the reagent pH is about 6.8 and at least about 25:1 when the reagent pH is about 7.4. (As with the liquid reagent, the ratio of divalent metal salt to phenazine derivative required to stabilize the film is higher at higher pHs because degradation of the phenazine derivative and the tetrazolium salt in the film, the fabric mesh, the glass fiber matrix, and the paper matrix is more pronounced at higher pHs. Also, the ratios would need to be higher to stabilize a film, a fabric mesh, a glass fiber matrix, and a paper matrix that included MTT. Stabilization of a film, a fabric mesh, a glass fiber matrix, and a paper matrix that included MTT would be easier under humidity conditions of less than about 15%.) Higher ratios of divalent metal salt to phenazine derivative may be used, but no further stabilization is achieved by such higher ratios.

Importantly, the inclusion of the divalent metal salts stabilizes the phenazine derivative/tetrazolium salt reagent at pHs from about 5.5 to about 7.8. (Such films that do not include a divalent metal salt show a formazan blank reaction upon drying the wet film for 15 minutes at about 50°C.) The reagent is stable without the inclusion of a divalent metal salt below pHs of about 5.5. However, above pHs of about 5.5 photochemical degradation of phenazine derivatives and autoreduction of tetrazolium salts become significant. The problem of degradation of phenazine derivatives and tetrazolium salts worsens as the pH increases. It is desirable to conduct assays at pH values above about 5.5 because many enzymes optimally function at pH values above about 5.5. For example, glucose dehydrogenase optimally functions at a pH of about 7.9, and hexokinase and glucose-6-phosphate dehydrogenase optimally function at a pH of about 7.8. Inclusion of the divalent metal salts of the present invention in a reagent make degradation of the phenazine derivative and tetrazolium salt insignificant in the pH range from 5.5 to about 7.8, thereby permitting better performance of assays in that pH range (where certain enzymes function best). Further, it is particularly important to stabilize films that include a phenazine derivative and a tetrazolium salt because degradation of the phenazine derivative and the tetrazolium salt is worsened by the drying process used to form the film.

A buffer is preferably provided in the reagent in order to provide a pH most beneficial to the assay. The buffer should be of sufficient type and in sufficient amount to provide and maintain a pH for the enzyme to function as a catalyst. Table 4 below exemplifies buffers that may be used in the pH range in which they effectively buffer a solution.

**TABLE 4**

| BUFFER | pH RANGE |
|---|---|
| MES(2-[N-Morpholino]ethanesulfonic acid) | 5.5-6.7 |
| BIS-TIS(bis[2-Hydroxyethyl]imino-tris-[hydroxymethyl]-methane) | 5.8-7.2 |
| ADA(N-[2-Acetamido]-2-iminodiacetic acid) | 6.0-7.2 |
| PIPES(Piperazine-N,N'-bis[2-ethanesulfonic acid)) | 6.1-7.5 |
| ACES(2-[(2-Amino-2-oxoethyl)-amino]ethanesulfonic acid) | 6.1-7.5 |
| BIS-TRIS PROPANE(1,3-bis[tris(Hydroxymethyl)methylamino]-propane) | 6.3-9.5 |
| MOPSO(3-[N-Morpholino]-2-hydroxy-propanesulfonic acid) | 6.2-7.6 |
| BES(N,N-bis[2-Hydroxyethyl]-2-aminoethanesulfonic acid) | 6.4-7.8 |
| MOPS(3-[N-Morpholino]propanesulfonic acid) | 6.5-7.9 |
| TES(N-tris[Hydroxymethyl]methyl-2-aminoethanesulfonic acid) | 6.8-8.2 |
| HEPES(N-2-Hydroxyethylpiperazine-N'-2-ethanesulfonic acid) | 6.8-8.2 |
| TAPSO(3-[N-tris(Hydroxymethyl) methylamino]-2-hydroxypropane-sulfonic acid) | 7.0-8.2 |
| POPSO(Piperazine-N,N'-bis[2-hydroxypropanesulfonic acid]) | 7.2-8.5 |
| EPPS(N[2-Hydroxyethyl]-piperazine-N'-3-propanesulfonic acid) | 7.3-8.7 |
| TRIS(tris[Hydroxymethyl)amino-methane) | 7.0-9.0 |
| TRICINE(N-tris[Hydroxymethyl]-methylglycine) | 7.4-8.8 |
| BICINE(N,N-bis[2-Hydroxyethyl] glycine) | 7.6-9.0 |
| TAPS(tris[Hydroxymethyl)methylamino-propanesulfonic acid) | 7.7-9.1 |

Examples of a liquid reagent and a film are given below.

### Reagent Example 1

A film may be prepared with the following ingredients in the following amounts (total mass of ingredients = 1 kilogram):

| **Ingredient** | **Amount in Grams** (Unless Otherwise Specified) . |
|---|---|
| 0.3 Molar (M) HEPES Buffer (pH = 7.5) | 253.3 |
| 20% (by weight (wt.)) aqueous composition of various polyoxyethylene ethers and other surface-active compounds, sold under the trademark TRITON X-100, available from Sigma Chemical Company | 14.5 |
| manganese sulfate | 16.7 (99 millimoles (mmol)) |
| nickel chloride | 23.6 (99 mmol) |
| diatomite, sold under the trademark CELATOM MW25, available from Eagle Picher | 157.4 |
| titanium dioxide, available from Kronos as TiO₂ RN 43 | 148.3 |
| phenazine ethosulfate, available from Research Organics, Inc. | 1.3 (3.9 mmol) |
| 3:2:1 (wt.:wt.:wt.) acetone:hexanol: methanol | 4.2 |
| 50:50 (wt.:wt.) water: polyvinylpropionate (sold under the trademark PROPIOFAN 70D, available from BASF) | 104.5 |
| 3% (by wt.) methyl cellulose, sold under the trademark TYLOSE MH 2000, available from Hoechst, in 0.3 M HEPES buffer | 261.9 |
| Glucose Oxidase (Grade I, available from Biozyme) | 1,754.4 kilounits |
| INT | 5.689(11.25 mmol) |

The film may be made as follows:
step # 1 - add 20% (by wt.) aqueous TRITON X-100 surfactant to the 0.3 M HEPES buffer (pH = 7.5);
step # 2 - next, add the manganese sulfate and nickel chloride;
step # 3 - next, add titanium dioxide and stir until the resulting mixture is homogeneous;
step # 4 - next, add CELATOM MW25 diatomite;
step # 5 - next, add 3% (by wt.) TYLOSE MH 2000 methyl cellulose in 0.3 M HEPES buffer;
step # 6 - next, add PROPIOFAN 70D;
step # 7 - next, add glucose oxidase;
step # 8 - next, add INT;
step # 9 - next, add 3:2:1 (wt.:wt.:wt.) acetone:hexanol:methanol;
step #10 - centrifuge to remove bubbles;
step #11 - next, stir until contents are homogeneous;
step #12 - next, filter the homogeneous contents through a mesh bag to remove clumps
step #13 - next, stir the filtrate until it is homogeneous;
step #14 - next, coat these contents onto Cronar plastic, providing a coating thickness of 250 micrometers (µm);
step #15 - dry the coating at 49° C for 30 minutes.

### Reagent Example 2

An aqueous reagent may be prepared with the following ingredients in the following concentrations:

| **Ingredient** | **Concentration (millimolar (mM) unless otherwise specified) .** |
|---|---|
| aqueous HEPES BUFFER (pH = 7.3) | 100 |
| magnesium chloride | 250 |
| phenazine ethosuifate | 5 |
| glucose oxidase | 1 kilounit/milliliter (ml) reagent |
| INT | 12 |

In preparing the above stated aqueous reagent, phenazine ethosulfate and INT should be added last to the reagent.

The assay of an analyte may be conducted by performing the following steps:
a. forming a test sample by combining a fluid sample
   containing the analyte and the reagent of the present invention;
b. incubating the test sample;
c. spectrophotometrically measuring the incubated test sample; and
d. correlating the spectrophotometric measurement to the concentration of analyte in the fluid sample.

The incubation temperature and time period for incubation will depend upon the assay being conducted. The wavelength of spectrophotometric measurement and the type of spectrophotometric measurement (for example absorbance, transmittance, or reflectance) will depend upon the tetrazolium salt utilized in the reagent and whether spectrophotometric measurements are made of a solution (utilizing a liquid reagent) or a film, respectively. Examples of specific assay procedures utilizing the specifically formulated reagents exemplified above are given below.

### ASSAYS FOR GLUCOSE

### Assay Example 1

In a cuvet, combine 2.9 milliliters (ml) of the above stated liquid reagent (Reagent Example 2) and 0.1 ml of a liquid unknown sample, containing an unknown amount of from about 0 to about 600 milligrams (mg) glucose per deciliter (dl) of sample, and vortex the resulting contents to mix the liquid unknown sample and liquid reagent. (assay step a. above) Incubate the resulting contents at room temperature for 15 seconds (assay step b. above); then read spectrophotometric absorbance of the incubated, resulting contents at 580 nanometers (nm). (assay step c. above) Compare spectrophotometric absorbance of the incubated, resulting contents with the spectrophotometric absorbance of a standard (that is, the above stated liquid reagent combined with and incubated, as specified above, with a liquid known sample containing a known amount of from about 0 to about 600 mg glucose per dl of known sample), and correlate the spectrophotometric absorbance of the incubated, resulting contents with the concentration of glucose in the liquid unknown sample. (assay step d. above)

### Assay Example 2

For glucose analysis performed with the film specified above (Reagent Example 1), add enough liquid unknown sample, containing an unknown amount of from about 0 to about 600 mg glucose per dl of sample, to wet the film. (assay step a. above) Incubate the wetted film for about 30 seconds at room temperature. (assay step b. above) Measure spectrophotometric reflectance of the incubated film at 580 nm. (assay step c. above) Compare measured spectrophotometric reflectance of the incubated film at 580 nm with the spectrophotometric reflectance of a film treated as above with the exception that a liquid known sample (containing a known amount of from about 0 to about 600 mg glucose per dl of sample) was used to wet the film rather than a liquid unknown sample, and correlate the spectrophotometric reflectance of the incubated film to the concentration of glucose in the unknown liquid sample.

The present invention has been disclosed in the above teachings with sufficient clarity and conciseness to enable one skilled in the art to make and use the invention, to know the best mode for carrying out the invention, and to distinguish it from other inventions and from what is old. Many variations and obvious adaptations of the invention will readily come to mind, and these are intended to be contained within the scope of the invention as claimed below.

## Claims

1. A reagent useful for the analysis of an analyte, comprising
an enzyme, a tetrazolium salt, a phenazine derivative that functions as an electron carrier and facilitates reduction of the tetrazolium salt, at least one divalent metal salt that does not function as an enzyme cofactor when the reagent is used for the analysis of the analyte, and water,
the enzyme being in sufficient amount to catalyze the reaction of analyte and phenazine derivative,
the phenazine derivative being in sufficient amount to catalyze the reduction of tetrazolium salt,
the tetrazolium salt being in sufficient amount to form a colored indicator when reduced, and
the divalent metal salt having a nickel, manganese, or magnesium cation and an inert anion and being in sufficient amount to stabilize the phenazine derivative and the tetrazolium salt.

2. The reagent of claim 1, further comprising:
a buffer in sufficient amount to provide and maintain a pH for the enzyme to function as a catalyst.

3. The reagent of claim 1 or 2, wherein the divalent metal salt is selected from the group consisting of nickel chloride, manganese sulfate, magnesium sulfate, magnesium chloride, and nickel sulfate.

4. A reagent useful for the analysis of an analyte, comprising:
an enzyme, a tetrazolium salt, a phenazine derivative that functions as an electron carrier and facilitates reduction of the tetrazolium salt, a divalent metal salt that does not function as an enzyme cofactor when the reagent is used for the analysis of the analyte, a buffer, and water,
the enzyme being in sufficient amount to catalyze the reaction of analyte and phenazine derivative,
the phenazine derivative being in sufficient amount to catalyze the reduction of tetrazolium salt,
the tetrazolium salt being in sufficient amount to form a colored indicator when reduced, the divalent metal salt is cobalt chloride in sufficient amount to stabilize the phenazine derivative and the tetrazolium salt, and
the buffer is tris(hydroxymethyl)aminomethane in sufficient amount to provide and maintain a pH for the enzyme to function as a catalyst.

5. The reagent of claims 1 to 3, wherein the divalent metal salt is selected from the group consisting of nickel chloride, manganese sulfate, and nickel sulfate.

6. The reagent of claims 1 to 5, wherein the molar ratio of divalent metal salt to phenazine derivative is at least about 30:1.

7. The reagent of claim 6, wherein the reagent pH is from about 5.5 to about 7.8.

8. A reagent that is incorporated into a film, a fabric mesh, a glass fibre matrix, or a paper matrix and useful for the analysis of an analyte from a fluid sample, the reagent comprising:
an enzyme, a tetrazolium salt, a phenazine derivative that functions as an electron carder, and facilitates reduction of the tetrazolium salt, and at least one divalent metal salt that does not function as an enzyme cofactor when the reagent is used for the analysis of the analyte,
the enzyme being in sufficient amount to catalyze the reaction of analyte and phenazine salt,
the phenazine derivative being in sufficient amount to catalyze the reduction of tetrazolium salt,
the tetrazolium salt being in sufficient amount to form a colored indicator when reduced, and
the divalent metal salt having a nickel, manganese, or magnesium cation and an inert anion and being in sufficient amount to stabilize the phenazine derivative and the tetrazolium salt.

9. The reagent of claim 8, further comprising:
a buffer in sufficient amount to provide and maintain a pH for the enzyme to function as a catalyst.

10. The reagent of claim 8 or 9, wherein the molar ratio of divalent metal salt to phenazine derivative is at least about 15:1.

11. The reagent of claims 8 to 10, wherein the molar ratio of divalent metal salt to phenazine derivative is at least about 25:1.

12. The reagent of claim 11, wherein the buffer is in sufficient amount to provide a pH from about 5.5 to about 7.8 when the fluid sample containing the analyte is added to the reagent.

13. The reagent of claims 8 to 12, wherein the divalent metal salt is selected from the group consisting of nickel chloride, manganese sulfate, magnesium sulfate, magnesium chloride, and nickel sulfate.

14. The reagent of claim 13, wherein the divalent metal salt is selected from the group consisting of nickel chloride, manganese sulfate, and nickel sulfate.

15. A method for the assay of an analyte, comprising the steps of:
a. forming a test sample by combining a fluid sample containing the analyte and the reagent of claims 1 to 14;
b. incubating the test sample;
c. spectrophotometrically measuring the incubated test sample; and
d. correlating the spectrophotometric measurement to the concentration of analyte in the fluid sample.

## Patentansprüche

1. Reagens, das zur Analyse eines Analyten brauchbar ist, umfassend:
ein Enzym, ein Tetrazolium-Salz, ein Phenazin-Derivat, das als Elektronenträger fungiert und die Reduktion des Tetrazolium-Salzes erleichtert, wenigstens ein Salz eines zweiwertigen Metalls, das nicht als Enzymcofaktor fungiert, wenn das Reagens zur Analyse des Analyten eingesetzt wird, und Wasser;
wobei das Enzym in ausreichender Menge vorliegt, um die Reaktion von Analyt und Phenazin-Derivat zu katalysieren;
wobei das Phenazin-Derivat in ausreichender Menge vorliegt, um die Reduktion des Tetrazolium-Salzes zu katalysieren;
wobei das Tetrazolium-salz in ausreichender Menge vorliegt, um einen farbigen Indikator zu bilden, wenn es reduziert wird; und
wobei das Salz des zweiwertigen Metalls ein Nickel-, Mangan-, oder Magnesiumkation und ein inertes Anion aufweist und in ausreichender Menge vorliegt, um das Phenazin-Derivat und das Tetrazolium-Salz zu stabilisieren.

2. Reagens nach Anspruch 1, des weiteren umfassend:
einen Puffer in ausreichender Menge, um einen pH für das Enzym bereitzustellen und aufrechtzuerhalten, so daß es als Katalysator wirkt.

3. Reagens nach Anspruch 1 oder 2, wobei das Salz des zweiwertigen Metalls ausgewählt ist aus der Gruppe bestehend aus Nickelchlorid, Mangansulfat, Magnesiumsulfat, Magnesiumchlorid und Nickelsulfat.

4. Reagens, das zur Analyse eines Analyten brauchbar ist, umfassend:
ein Enzym, ein Tetrazolium-Salz, ein Phenazin-Derivat, das als Elektronenträger fungiert und die Reduktion des Tetrazolium-Salzes erleichtert, ein Salz eines zweiwertigen Metalls, das nicht als Enzymcofaktor fungiert, wenn das Reagens zur Analyse des Analyten eingesetzt wird, einen Puffer und Wasser;
wobei das Enzym in ausreichender Menge vorliegt, um die Reaktion von Analyt und Phenazin-Derivat zu katalysieren;
wobei das Phenazin-Derivat in ausreichender Menge vorliegt, uni die Reduktion des Tetrazolium-Salzes zu katalysieren;
wobei das Tetrazolium-Salz in ausreichender Menge vorliegt, um einen farbigen Indikator zu bilden, wenn es reduziert wird, das Salz des zweiwertigen Metalls Cobaltchlorid in ausreichender Menge ist, uni das Phenazin-Derivat und das Tetrazoliuin-Salz zu stabilisieren; und
der Puffer Tris(hydroxymethyl)aminomethan in ausreichender Menge ist, um einen pH für das Enzym bereitzustellen und aufrechtzuerhalten, so daß es als Katalysator wirkt.

5. Reagens nach den Ansprüchen 1 bis 3, wobei das Salz des zweiwertigen Metalls ausgewählt ist aus der Gruppe bestehend aus Nickelchlorid, Mangansulfat und Nickelsulfat.

6. Reagens nach den Ansprüchen 1 bis 5, wobei das Molverhältnis von Salz des zweiwertigen Metalls zu Phenazin-Derivat wenigstens etwa 30:1 beträgt.

7. Reagens nach Anspruch 6, wobei der pH des Reagens etwa 5,5 bis etwa 7,8 beträgt.

8. Reagens, das in einen Film, ein Gewebenetz, eine Glasfasermatrix oder in eine Papierinatrix eingebracht und für die Analyse eines Analyten aus einer flüssigen Probe brauchbar ist, umfassend:
ein Enzym, ein Tetrazolium-Salz, ein Phenazin-Derivat, das als Elektronenträger fungiert und die Reduktion des Tetrazolium-Salzes erleichtert, sowie wenigstens ein Salz eines zweiwertigen Metalls, das nicht als Enzymcofaktor fungiert, wenn das Reagens zur Analyse des Analyten eingesetzt wird;
wobei das Enzym in ausreichender Menge vorliegt, um die Reaktion von Analyt und Phenazin-Derivat zu katalysieren;
wobei das Phenazin-Derivat in ausreichender Menge vorliegt, um die Reduktion des Tetrazolium-Salzes zu katalysieren;
wobei das Tetrazolium-Salz in ausreichender Menge vorliegt, um einen farbigen Indikator zu bilden, wenn es reduziert ist; und
wobei das Salz des zweiwertigen Metalls ein Nickel-, Mangan-, oder Magnesiumkation und ein inertes Anion aufweist und in ausreichender Menge vorliegt, um das Phenazin-Derivat und das Tetrazolium-Salz zu stabilisieren.

9. Reagens nach Anspruch 8, des weiteren umfassend:
einen Puffer in ausreichender Menge, um einen pH für das Enzym bereitzustellen und aufrechtzuerhalten, so daß es als Katalysator wirkt.

10. Reagens nach Anspruch 8 oder 9, wobei das Molverhältnis von Salz des zweiwertigen Metalls zu Phenazin-Derivat wenigstens etwa 15:1 beträgt.

11. Reagens nach den Ansprüchen 8 bis 10, wobei das Molverhältnis von Salz des zweiwertigen Metalls zu Phenazin-Derivat wenigstens etwa 25:1 beträgt.

12. Reagens nach Anspruch 11, wobei der Puffer in ausreichender Menge vorliegt, um einen pH von etwa 5,5 bis etwa 7,8 zu ergeben, wenn die den Analyten enthaltende flüssige Probe dem Reagens zugesetzt wird.

13. Reagens nach den Ansprüchen 8 bis 12, wobei das Salz des zweiwertigen Metalls ausgewählt ist aus der Gruppe bestehend aus Nickelchlorid, Mangansulfat, Magnesiumsulfat, Magnesiumchlorid und Nickelsulfat.

14. Reagens nach Anspruch 13, wobei das Salz des zweiwertigen Metalls ausgewählt ist aus der Gruppe bestehend aus Nickelchlorid, Mangansulfat und Nickelsulfat.

15. Verfahren zum Testen auf einen Analyten, umfassend die Schritte:
a) Bilden einer Testprobe durch Vereinigen einer den Analyten enthaltenden flüssigen Probe mit dem Reagens nach den Ansprüchen 1 bis 14;
b) Inkubieren der Testprobe;
c) spektrophotometrische Messung der inkubierten Testprobe; und
d) Korrelieren der spektrophotometrischen Messung mit der Konzentration des Analyten in der flüssigen Probe.

## Revendications

1. Réactif utile pour l'analyse d'un analyte, comprenant
une enzyme, un sel de tétrazolium, un dérivé de phénazine qui fonctionne comme transporteur d'électrons et qui facilite la réduction du sel de tétrazolium, au moins un sel métallique divalent qui ne fonctionne pas comme cofacteur d'enzyme lorsque le réactif est utilisé pour l'analyse de l'analyte, et de l'eau, l'enzyme étant présente en quantité suffisante pour catalyser la réaction de l'analyte et du dérivé de phénazine,
le dérivé de phénazine étant présent en quantité suffisante pour catalyser la réduction du sel de tétrazolium,
le sel de tétrazolium étant présent en quantité suffisante pour former un indicateur coloré, lorsqu'il est réduit, et
le sel métallique divalent présentant un cation de nickel, de manganèse ou de magnésium et un anion inerte et étant présent en quantité suffisante pour stabiliser le dérivé de phénazine et le sel de tétrazolium.

2. Réactif selon la revendication 1, comprenant en outre :
un tampon en quantité suffisante pour donner et maintenir un pH pour que f'enzyme fonctionne comme catalyseur.

3. Réactif selon la revendication 1 ou 2, dans lequel le sel métallique divalent est choisi dans le groupe constitué du chlorure de nickel, du sulfate de manganèse, du sulfate de magnésium, du chlorure de magnésium et du sulfate de nickel.

4. Réactif utile pour l'analyse d'un analyte, comprenant :
une enzyme, un sel de tétrazolium, un dérivé de phénazine qui fonctionne comme transporteur d'électrons et qui facilite la réduction du sel de tétrazolium,
un sel métallique divalent qui ne fonctionne pas comme cofacteur d'enzyme lorsque le réactif est utilisé pour l'analyse de l'analyte, un tampon et de l'eau,
l'enzyme étant présente en quantité suffisante pour catalyser la réaction de l'analyte et du dérivé de phénazine,
le dérivé de phénazine étant présent en quantité suffisante pour catalyser la réduction du sel de tétrazolium,
le sel de tétrazolium étant présent en quantité suffisante pour former un indicateur coloré, lorsqu'il est réduit,
le sel métallique divalent est le chlorure de cobalt en quantité suffisante pour stabiliser le dérivé de phénazine et le sel de tétrazolium, et
le tampon est le tris(hydroxyméthyl)aminométhane en quantité suffisante pour donner et maintenir un pH pour que l'enzyme fonctionne comme catalyseur.

5. Réactif selon les revendications 1 à 3, dans lequel le sel métallique divalent est choisi dans le groupe constitué du chlorure de nickel, du sulfate de manganèse et du sulfate de nickel.

6. Réactif selon les revendications 1 à 5, dans lequel le rapport molaire du sel métallique divalent au dérivé de phénazine est d'au moins environ 30 : 1.

7. Réactif selon la revendication 6, dans lequel le pH du réactif vaut d'environ 5,5 à environ 7,8.

8. Réactif qui est incorporé dans un film, dans les mailles d'un tissu, dans une matrice de fibres de verre ou dans une matrice en papier et qui est utile pour l'analyse d'un analyte d'un échantillon fluide, réactif comprenant une enzyme, un sel de tétrazolium, un dérivé de phénazine qui fonctionne comme transporteur d'électrons et qui facilite la réduction du sel de tétrazolium, et au moins un sel métallique divalent qui ne fonctionne pas comme cofacteur d'enzyme lorsque le réactif est utilisé pour l'analyse de l'analyte,
l'enzyme étant présente en quantité suffisante pour catalyser la réaction de l'analyte et du sel de phénazine,
le dérivé de phénazine étant présent en quantité suffisante pour catalyser la réduction du sel de tétrazolium,
le sel de tétrazolium étant présent en quantité suffisante pour former un indicateur coloré, lorsqu'il est réduit, et
le sel métallique divalent présentant un cation de nickel, de manganèse ou de magnésium et un anion inerte et étant présent en quantité suffisante pour stabiliser le dérivé de phénazine et le sel de tétrazolium.

9. Réactif selon la revendication 8, comprenant en outre :
un tampon en quantité suffisante pour donner et maintenir un pH pour que l'enzyme fonctionne comme catalyseur.

10. Réactif selon la revendication 8 ou 9, dans lequel le rapport molaire du sel métallique divalent au dérivé de phénazine est d'au moins environ 15 : 1.

11. Réactif selon les revendications 8 à 10, dans lequel le rapport molaire du sel métallique divalent au dérivé de phénazine est d'au moins environ 25 : 1.

12. Réactif selon la revendication 11, dans lequel le tampon est présent en une quantité suffisante pour donner un pH d'environ 5,5 à environ 7,8 lorsque l'échantillon fluide contenant 'analyte est ajouté au réactif.

13. Réactif selon les revendications 8 à 12, dans lequel le sel métallique divalent est choisi dans le groupe constitué du chlorure de nickel, du sulfate de manganèse, du sulfate de magnésium, du chlorure de magnésium et du sulfate de nickel.

14. Réactif selon la revendication 13, dans lequel le sel métallique divalent est choisi dans le groupe constitué du chlorure de nickel, du sulfate de manganèse et du sulfate de nickel.

15. Méthode pour le dosage d'un analyte, comprenant les étapes de :
a. la formation d'un échantillon à analyser par combinaison d'un échantillon fluide contenant l'analyte et le réactif selon les revendications 1 à 14 ;
b. l'incubation de l'échantillon à analyser ;
c. la mesure spectrophotométrique de l'échantillon à analyser incubé ; et
d. la corrélation entre la mesure spectrophotométrique et la concentration de l'analyte dans l'échantillon fluide.
